Europäisches Patentamt

European Patent Office

Office européen des brevets

(19)

(11) Publication number: **0 256 707 B1**

# EUROPEAN PATENT SPECIFICATION

(12)

(45) Date of publication of the patent specification: 28.11.90

(51) Int. Cl.⁵: **C07C 2/00, C10G 31/00**

(21) Application number: **87306688.0**

(22) Date of filing: **29.07.87**

(54) **Catalytic process for oligomerizing ethene.**

(30) Priority: **05.08.86 US 893522**
**05.09.86 US 903915**

(43) Date of publication of application:
**24.02.88 Bulletin 88/8**

(45) Publication of the grant of the patent:
**28.11.90 Bulletin 90/48**

(84) Designated Contracting States:
**AT BE DE FR GB IT NL SE**

(56) References cited:
**EP-A- 0 030 431**
**EP-A- 0 133 052**
**EP-A- 0 159 848**
**US-A- 3 689 589**
**US-A- 4 542 251**

(73) Proprietor: **MOBIL OIL CORPORATION, 150 East 42nd Street, New York New York 10017(US)**

(72) Inventor: **Kushnerick, John Douglas, 248 Springhouse Lane, Media Pennsylvania 19063(US)**
Inventor: **Krambeck, Frederick John, 108 Greenvale Road, Cherry Hill New Jersey 08034(US)**
Inventor: **Tabak, Samuel Allen, 204 East Pine Street, Wennonah New Jersey 08090(US)**
Inventor: **Garwood, William Everett, 125 Warwick Road, Haddonfield New Jersey 08033(US)**

(74) Representative: **Colmer, Stephen Gary, Patent Department c/o Mobil Services Company Limited Mobil Court 3 Clements Inn, London WC2A 2EB(GB)**

## Description

This invention relates to a catalytic technique for upgrading an ethene-containing lower olefin to a heavier liquid hydrocarbon product.

Recent developments in zeolite catalysis and hydrocarbon conversion processes have created interest in utilizing olefinic feedstocks for producing $C_5^+$ gasoline and diesel fuel. For example, U.S. Patent Nos. 3 960 978 and 4 021 502 disclose conversion of $C_2$-$C_5$ olefins, alone or in admixture with paraffinic components, into higher hydrocarbons over crystalline zeolites having controlled acidity. In addition, U.S. Patent No. 4 542 251 discloses a process for oligomerizing $C_2$ to $C_{20}$ olefins over nickel-containing ZSM-5 and/or ZSM-11, but teaches that the feed should be low in water.

In particular it has been shown that HZSM-5 is effective for oligomerizing lower olefins, especially propene and butenes, at moderately elevated temperatures and pressures to provide products useful as liquid fuels, especially $C_5^+$ aliphatic and aromatic hydrocarbons. Product distribution for the liquid hydrocarbons can be varied by controlling process conditions, such as temperature, pressure and space velocity. Gasoline ($C_5$-$C_9$) is readily formed at elevated temperature (e.g., up to about 400°C) and moderate pressure from ambient to 5500 kPa, preferably 250 to 2900 kPa. Olefinic gasoline can be produced in good yield and may be recovered as a product or fed to a low severity, high pressure reactor system for further conversion to heavier distillate-range products. Distillate mode operation can be employed to maximize production of $C_{10}^+$ aliphatics by reacting the lower and intermediate olefins at high pressure and moderate temperature. Operating details for typical oligomerization units are disclosed in U.S. Patents 4 456 779; 4 497 968 and 4 433 185. It is, however, found that the low severity distillate mode conditions do not convert a major fraction of ethene. Thus, while propene, butene-1, and others may be converted to the extent of 50% to 95% in the moderate temperature distillate mode, only 10% to 30% of the ethene component will be converted using HZSM-5 or similar acid zeolites. This is a problem since many olefinic feedstocks of commercial interest, such as FCC offgas, contain significant proportions of ethene. An object of the present invention is therefore to provide an improved method for converting ethene-containing lower olefinic feedstocks to heavier liquid hydrocarbon products.

According to the invention, there is provided a catalytic process for converting an ethene-containing lower olefinic feedstock to a heavier liquid hydrocarbon product, comprising the step of contacting the ethene-containing feedstock at an elevated temperature with at least one conversion catalyst comprising a nickel ethene oligomerization component and a shape-selective medium pore acidic zeolite component to convert at least a portion of the lower olefinic component to heavier hydrocarbons, water being coted with said feedstock during the contacting step thereby maintaining the nickel component in an oxidized state.

The ethene conversion process of the invention may comprise the primary or a secondary step in a light olefin oligomerization scheme. Thus in one preferred embodiment, there is provied a continuous multi-stage catalytic process for converting an ethene-containing lower olefinic feedstock to heavier liquid hydrocarbon product, comprising the steps of contacting said feedstock at elevated temperature in a primary stage reaction zone with a first catalyst comprising a shape selective medium pore zeolite to convert at least a portion of the lower olefinic components to intermediate olefinic hydrocarbons; cooling the primary stage reaction effluent by introducing a stream of cold water sufficient to reduce the primary stage effluent to second stage reaction temperature; and contacting unreacted ethene, water and at least a portion of the intermediate olefinic hydrocarbons from the primary stage effluent with a nickel-containing shape selective medium pore zeolite catalyst at an elevated temperature to produce a secondary stage reaction effluent comprising gasoline and/or distillate range hydrocarbons; said water being present in amount sufficient to maintain the nickel in an oxidized state.

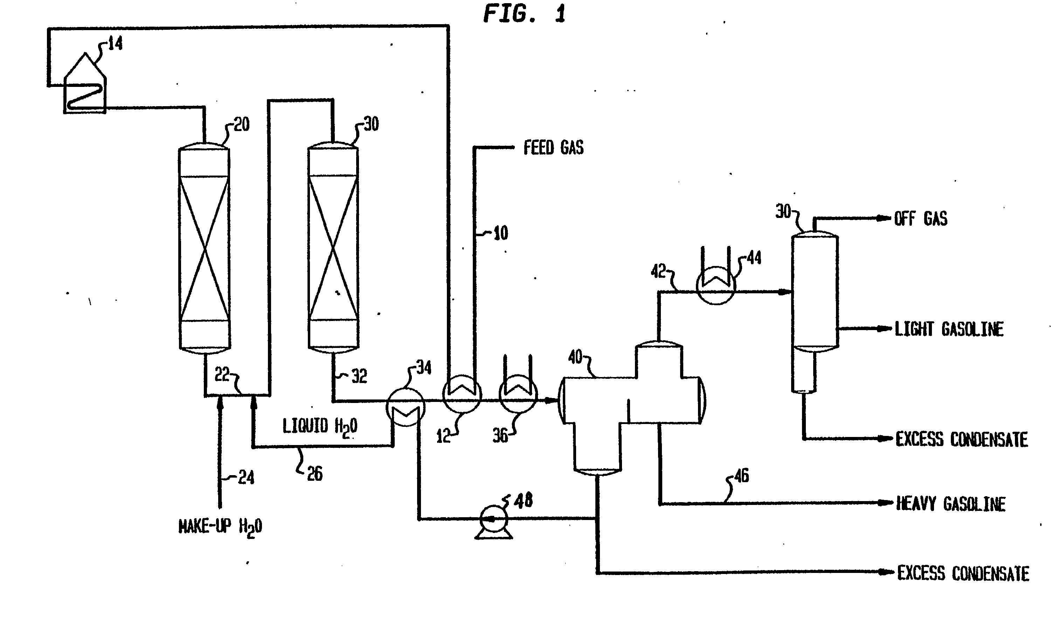

In the accompanying drawings, Fig. 1 is a schematic diagram depicting a two-stage process according to the present invention;

Fig. 2 is a graphic plot of ethene conversion using different catalysts;
Figs. 3 and 4 plot reactor temperature against time; and
Fig. 5 shows the effects of water cofeeding.

The catalyst material used in the ethene conversion process of the invention includes two catalytic components: (1) a metallic oligomerization component defined by ionic $Ni^{+2}$, and (2) a shape-selective medium pore acidic oligomerization catalyst, such as ZSM-5. These components may be present in admixture or combined in a unitary bifunctional solid particle. In a preferred embodiment, a nickel ion-exchanged zeolite, such as Ni-ZSM-5, is employed; however, it is possible to use a nickel-impregnated zeolite or a catalyst composed of an admixture of a zeolite with a nickel (impregnated or exchanged) containing support.

The medium pore (i.e., about 5-7A) shape-selective crystalline zeolites used herein preferably have a silica-to-alumina molar ratio of at least 12 and a constraint index of 1 to 12. Representative zeolites include ZSM-5, ZSM-11, ZSM-12, ZSM-23 and ZSM-35. ZSM-5 is disclosed in U.S. Patent No. 3,702,886 and U.S. Patent No. Re. 29,948; ZSM-11 is disclosed in U.S. Patent 3,709,979; ZSM-12 is disclosed in U.S. Patent 3,832,449; ZSM-23 is disclosed in U.S. Patent 4,076,979 and ZSM-35 is disclosed in U.S. Patent 4,016,245. Preferably the zeolite has an acid cracking activity, alpha, of about 10-250.

## ETHENE CONVERSION AS THE PRIMARY OLEFIN OLIGOMERIZATION STAGE

Where the ethene conversion process of the invention is effected as the primary stage in an olefin oligomerization process, the preferred feedstock comprises $C_2$-$C_6$ olefins including at least 5 mole % ethene. Non-deleterious components, such as paraffins and inert gases, may be present. A particularly useful feedstock is a light gas by-product of FCC gas oil cracking units containing typically 10-40 mol % $C_2$-$C_4$ olefins and 5-35% $H_2$ with minor amounts of $C_1$-$C_4$ paraffins and $N_2$.

In a typical process, the ethylene-rich $C_2^+$ olefinic feedstock is converted in a catalytic reactor under oligomerization conditions and moderate pressure to produce $C_5^+$ hydrocarbons rich in gasoline-range olefins and aromatics. Light gas, including unreacted ethylene, may be recovered from the product stream in a separation unit and recycled to the reactor, while up to a major amount of condensed $C_5^+$ hydrocarbons may be cascaded from the primary stage into a second distillate mode reactor. This will advantageously maximize distillate production by polymerizing gasoline boiling-range components. Because the primary ethene conversion stage is preferably operated at a pressure level of 200 - 3600 kPa, the compression requirements are efficient. A condensed intermediate liquid stream may be brought to higher pressure in a second catalytic reactor zone by pumping prior to contacting a second catalyst bed for further conversion to $C_{10}^+$ distillate range product. Gasoline rich in $C_5$-$C_9$ olefins may be recovered from the second separation zone or recycled to the distillate mode reactor zone.

A typical primary ethene reactor unit employs an adiabatic catalyst zone with adjustable gas recycle, whereby the reaction exotherm can be carefully controlled to prevent excessive temperature rise above the usual operating range of 100°C. to 450°C., preferably at average reactor temperature of 250°C. to 400°C. Energy conservation in the system utilizes at least a portion of the reactor exotherm heat value by exchanging hot reactor effluent from one or more reactors with feedstock and/or recyle stream. Optional heat exchangers may recover heat from the effluent stream prior to fractionation. It is preferred to operate the ethene conversion reactor at moderate pressure of 200 to 3600 kPa (15-500 psig).

The reactor system may contain multiple downflow adiabatic catalytic zones in each reactor vessel. The weight hourly space velocity (WHSV, based on total olefins in the fresh feedstock) is preferably 0.1-2 WHSV. In this mode, the molar recycle ratio for light gas is at least equimolar, based on total olefins in the fresh feedstock. The preferred molar ratio of recycle to fresh feedstock olefin is at least 2:1. Typical product fractionation systems are described in U.S. Patents 4,456,779 and 4,504,693.

## ETHENE CONVERSION AS A SECONDARY OLEFIN OLIGOMERIZATION STAGE

Where the ethene conversion process of the invention is effected as a secondary stage in an olefin oligomerization sequence, the preferred feedstock comprises at least 10 mole % ethene and may consist essentially of $C_2$-$C_6$ olefins, such normal mono-olefins and isomers thereof,. and reducing gas, such as hydrogen and CO. Non-deleterious components, such as paraffins, may also be present.

Referring to Fig. 1, a typical process flowsheet is shown wherein the ethylene-rich $C_2^+$ olefinic feedstock is converted in a multi-stage reactor system under oligomerization conditions and moderate pressure to produce intermediate range $C_5^+$ hydrocarbons rich in gasoline range olefins. Gaseous feedstock, such as by-product light gas from cracking of petroleum fractions, is introduced through conduit 10 under process pressure, preheated in exchanger 12 and furnace 14, and contacted with oligomerization catalyst in primary reactor 20 under adiabatic conditions to convert predominantly the $C_3^+$ olefin components. The primary stage effluent stream 22 is mixed with a liquid coolant injected via makeup line 24 and recycle line 26. The cooled intermediate stream, rich in ethene and intermediate olefinic hydrocarbons is passed at the desired temperature to the secondary stage reactor 30, where the cascaded stream is contacted with a Ni-ZSM-5 oligomerization catalyst or mixture in the presence of water to convert at least a significant portion of the ethene. The secondary stage effluent stream 32 is then cooled by heat exchangers 34, 12, 36 to condense an aqueous phase and heavy gasoline range liquid hydrocarbons, rich in $C_9^+$ olefins and aromatics. Phase separation unit 40 may be operated advantageously by adjusting pressure and temperature to fractionate the hydrocarbons into a light gasoline containing vapor stream 42, which is cooled by exchanger 44 prior to additional fractionation in distilation tower 50 to recover a $C_5$-$C_8$ light gasoline product, $C_1$-$C_4$ offgas containing hydrogen, etc. A heavy gasoline stream 46, rich in $C_9^+$ hydrocarbons may be blended as fuel stock or further refined in a known manner. The aqueous liquid stream, consisting essentially of water and dissolved oxygenates, is recycled under control of pump 48, exchanger 34 and conduit 26 to the primary stage. Excess condensate may be withdrawn or makeup water added, as required to meet the thermodynamic requirements or catalyst activation needs of the system.

In the system described above, a typical FCC offgas can be converted in the primary reactor starting at about 205°C. (400°F.) at the start of fresh catalyst cycle, with adiabatic heat of reaction resulting in a temperature rise of about 75°C. (135°F.) across the primary conversion zone. Injected water at a rate of about 8 parts by weight per part of hydrocarbon reduces the interstage effluent stream to about 230°C.

3

(450°F.) for the secondary reactor, wherein the high exothermic heat generated by ethene conversion results in a temperature rise of about 135°C. (240°F.) to a final stream temperature of about 365°C. (690°F). Other reaction conditions are given in the following description..

a)Stage I - Primary Oligomerization Reactor Operation

The initial olefin upgrading stage is effected in known manner over a medium pore shape selective zeolite oligomerization catalyst, such as HZSM-5, for converting a major amount of the $C_3^+$ olefinic hydrocarbons in the feedstock to liquid hydrocarbons comprising $C_5^+$. Product may be recovered by a fractionation system as described in U.S. Patents 4,456,779 and 4,504,693 (Owen, et al.).

The entire primary stage effluent stream including unreacted ethene, propene, butenes and amount of $C_5^+$ hydrocarbons from the primary stage may then be cascaded into the secondary stage reactor, along with injected quench water. This will optimize the process and will maximize liquid production by polymerizing the lower boiling range components. However, interstage separators may be employed between stages to effect preliminary product separation and/or provide recycle.

b) Stage II- Ethene Reactor Operation

In the secondary stage depicted in Fig. 1, the combination of catalyst and process conditions should be effective to convert a major amount (more than 50%) of ethene. This degree of reactivity may be achieved by elevated temperature, catalyst activity and space velocity to achieve the required degree of reaction severity. Ethene or other unreacted gaseous components may be present in the interstage cascade stream with hydrogen, carbon oxides, methane, nitrogen or other inert gases.

A typical high severity multi-zone reactor system employs inter-zone quench, whereby the reaction exotherm can be controlled to prevent excessive temperature rise above the normal moderate range of 100° to 450°C., preferably 250°C. to 400°C. Advantageously, the space velocity (LHSV based on olefin feed) is 0.5 to 1. Heat exchangers provide cooling and reduce the effluent to fractionation temperature. Heat exchangers may recover heat from the effluent stream prior to fractionation. It is preferred to operate the reactors at moderate pressure of 200 to 2900 kPa (15–400 psig).

The reactor system may contain multiple downflow adiabatic catalytic zones in a single reactor vessel. The weight hourly space velocity (WHSV, based on total fresh feedstock) is 0.1–2 LHSV. In this system recycle ratio of light gas is not necessary, and the cost of recompressing a recycled diluent is avoided.

The invention will now be more particulary described with reference to the Examples

EXAMPLES 1–3 (COMPARATIVE)

In order to demonstrate the improvement in ethene conversion using a bifunctional catalyst, a series of comparative runs were conducted in a fixed bed tubular reactor using HZSM-5 and NiZSM-5. The unexchanged HZSM-5 catalyst had an alpha value of about 180, a silica-to-alumina ratio of 70:1, and crystalline size of about 0.02 to 0.05 microns. The catalyst was prepared as an extrudate with 35 wt.% alumina binder. The NiZSM-5 was produced from the calcined acid form of HZSM-5 by ion-exchange with nickel ($Ni^{+2}$) followed by recalcination to produce a bifunctional catalyst containing about 1 wt.% Ni.

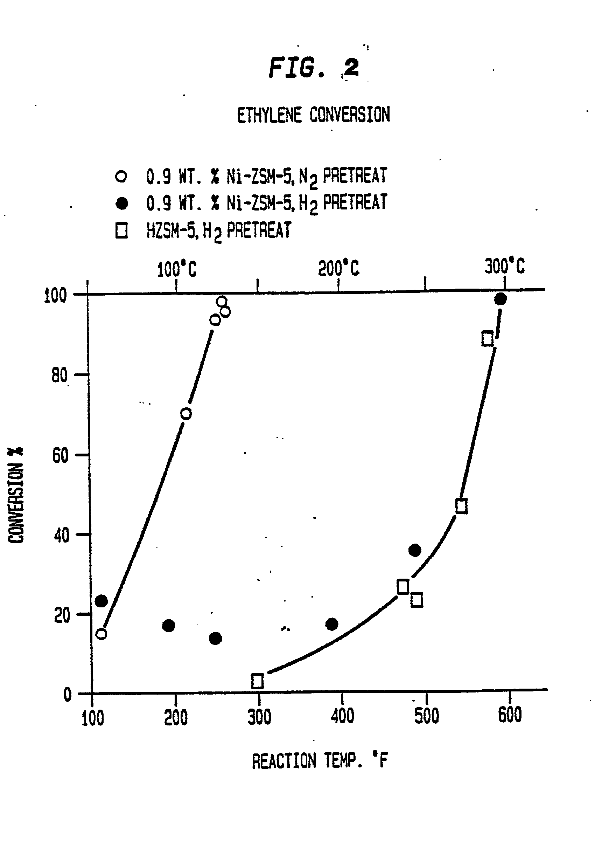

To show how the preferred nickel-treated ZSM-5 catalyst is affected by metal valence state, comparative runs are made in which the nickel ion-exchanged ZSM-5 was treated in a reducing atmosphere. The graphic plot in FIG. 2 compares conversion of ethylene over HZSM-5 0.9% Ni-ZSM-5 (ion exchanged) and reduced nickel catalyst. The reduced nickel ($Ni^o$) was produced by calcining the exchanged Ni-ZSM-5 at 480°C. in hydrogen instead of nitrogen, thus providing a material in which the major amount of nickel was present in the reduced metallic state. While the reduced catalyst had significant activity initially, the activity decreased rapidly as temperature was increased during the run, approaching the lower activity of HZSM-5. Since the feedstock used in the process of the invention will normally contain a reducing gas, particularly hydrogen, the presence of water in the feedstock is necessary to maintain the $Ni^{2+}$ in the ethene conversion catalyst in the oxidized state. Typically water is present in an amount comprising at least 0.1, preferably 0.3–2, moles water/mole of hydrogen.

The conversion of ethene ($C_2^=$) using HZSM-5 catalyst requires excessively high temperature, above 280°C., to obtain more than 50% conversion, thus increasing aromatics yield. By contrast, the acidic Ni-ZSM-5 bifunctional catalyst converted a major amount of ethene at moderate temperature.

EXAMPLES 4 - 6

Continuous runs were made in a larger scale reactor unit employing standard ZSM-5 (65%) extrudate catalyst at elevated pressure. Example 4 (Comparative) employed HZSM-5 having an acid cracking activity (alpha value) of about 200. Examples 5 and 6 employed nickel-exchanged (0.9 wt.%) acid ZSM-5. The fresh feedstock for Examples 4 and 6 contained 12.6 mol % ethene ($C_2^=$), 7.9% propene ($C_3^=$), 53.6% $N_2$ and 25.9% $H_2$. In Example 5, the propene was replaced with $N_2$ (total of 61.5% $N_2$ in feed). These experimental runs were conducted at similar conversion rates and the results are set forth in Table I.

| TABLE I | | | |
|---|---|---|---|
| Example | 4 | 5 | 6 |
| Days on stream | 7.5 | 7.3 | 15.5 |
| OPERATING CONDITIONS | | | |
| WHSV on Hydrocarbon feed, 1/hr | 1.0 | 0.98 | 0.56 |
| Reactor Pressure, KPA | 1825 | 1825 | 3204 |
| Gas recycle ratio, MOL/MOL | 2.0 | 2.0 | 2.0 |
| Average reactor temp., °C | 376 | 261 | 286 |
| $C_2$= Partial pressure at Reactor inlet, KPA | 87.6 | 106.4 | 165.0 |
| $C_3$= Partial pressure at Reactor inlet, KPA | 33.6 | 49.4 | 4.1 |
| Mole $H_2O$/mole $H_2$ at reactor inlet | 0.0 | 0.3 | 0.3 |
| Propane/Propene Ratio (RI) | 1.13 | 1.14 | 1.20 |
| | | | |
| YIELDS ON HYDROCARBON, WT% | | | |
| $C_5$+ including alkylate | 82.3 | 75.4 | 72.1 |
| potential alkylate | 9.3 | 9.4 | 10.4 |
| $C_4$+ | 86.1 | 79.2 | 73.1 |
| $C_5$+ | 73.0 | 66.0 | 61.7 |
| $C_5$'s | 14.8 | 13.3 | 10.8 |
| $NC_4$ | 1.7 | 1.7 | 1.6 |
| $IC_4$ | 4.8 | 4.8 | 5.4 |
| $C_4$= | 6.6 | 6.7 | 4.3 |
| $C_3$ | 2.8 | 2.4 | 2.3 |
| $C_3$= | 2.5 | 2.1 | 1.9 |
| $C_2$ | 0.6 | 7.1 | 10.4 |
| $C_2$= | 8.0 | 9.2 | 12.3 |
| $C_1$ | 0.1 | 0.0 | 0.0 |
| | | | |
| CONVERSION, WT% | | | |
| $C_2$= | 83.7 | 84.0 | 87.7 |
| $C_3$= | 94.7 | 95.2 | 0.0 |
| Total feed olefin | 89.0 | 88.9 | 87.7 |
| | | | |
| PRODUCT PROPERTIES | | | |
| Raw octane, R+O | 93.1 | — | 89.8 |
| Raw octane, M+O | — | — | — |
| Specific Gravity at 15.6°C | 0.734 | 0.736 | 0.750 |
| | | | |
| Boiling Point DISTRIBUTION, °C | | | |
| 5 | — | 18 | 22 |
| 10 | — | 35 | 40 |
| 30 | — | 73 | 91 |
| 50 | — | 109 | 123 |
| 70 | — | 139 | 151 |
| 90 | — | 181 | 188 |
| 95 | — | 201 | 201 |
| 99.5 | — | 268 | 263 |

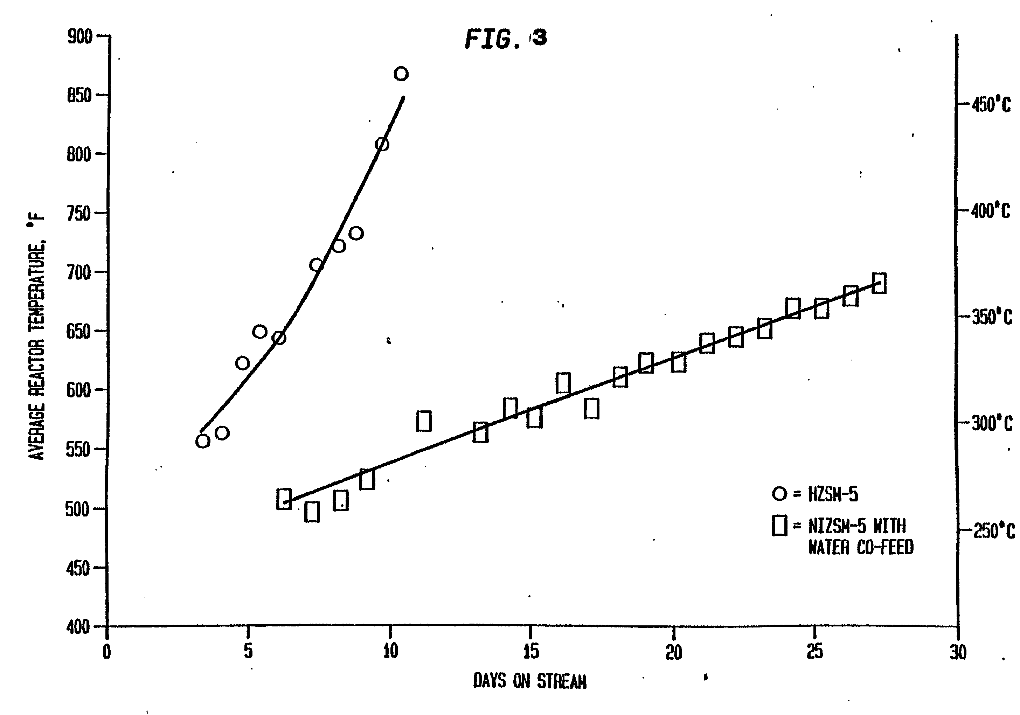

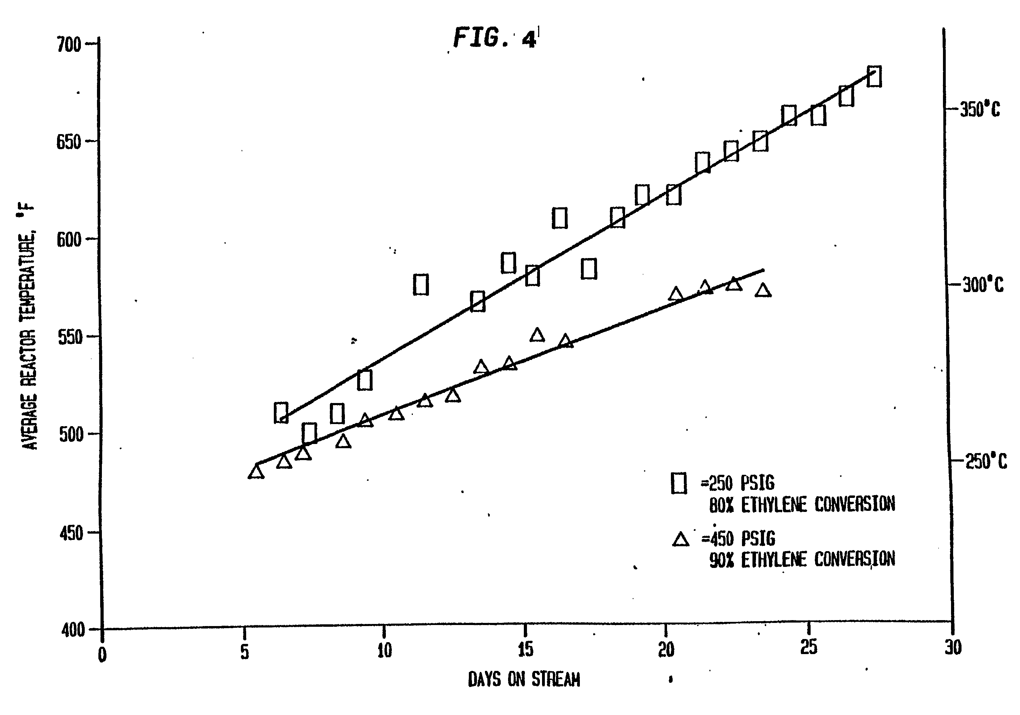

These examples are depicted graphically in FIGS. 3 and 4 for long-term, continuous runs. FIG. 3 shows the advantage of NiZSM-5 with water cofeed over HZSM-5, with catalyst deactivation rate being reduced by about a factor of 5. These average reactor temperatures are normalized to 80% ethylene conversion. FIG. 4 shows the advantage of operating at increased pressures. By raising total pressure from 1825 kPa (250 psig) to 3200 kPa (450 psig), it is possible to operate at 10% higher ethylene conversion and still further reduce catalyst deactivation rate by about a factor of 1.5. The average reactor temperatures for the 3200 kPa experiment have been normalized to a constant 90% ethylene conversion.

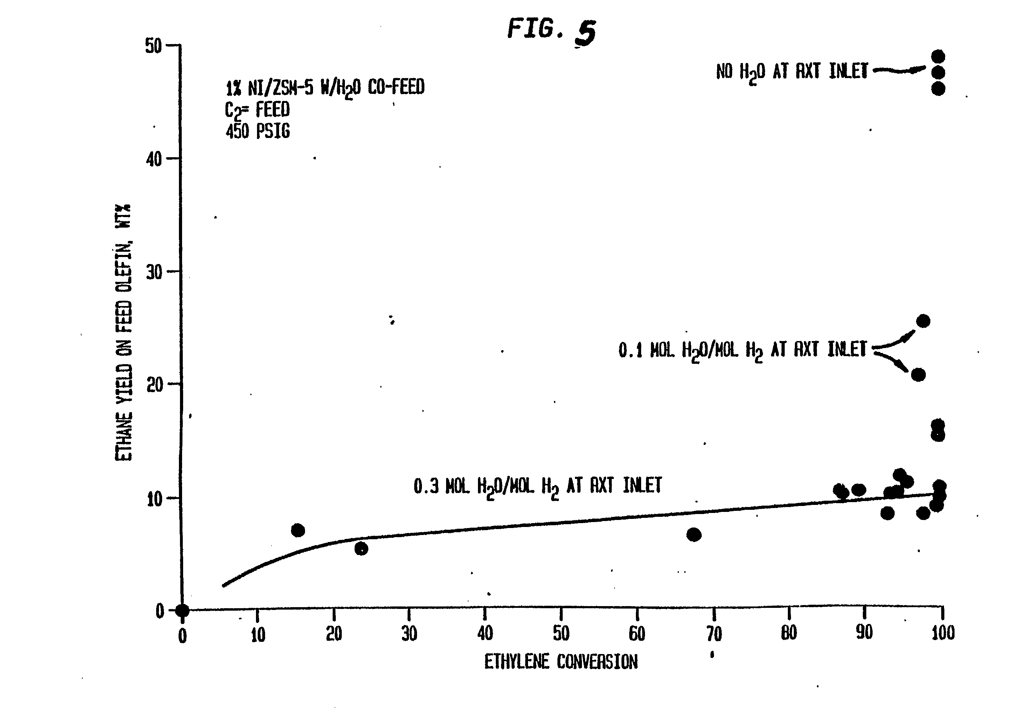

FIG. 5 graphically depicts the effect of water cofeeding on selectivity in Example 6. This plot shows that with NiZSM-5, water cofeed is required to maintain selectivity to higher molecular weight product. Without the water, ethylene hydrogenation becomes significant. These data are set forth in Table II:

## TABLE II

### Reversible Effect of Water Removal

#### Example 6

| | With $H_2O$ | $H_2O$ Removed | $H_2O$ Restored |
|---|---|---|---|
| Time on stream (days) | 16.5 | 17.5 | 20.5 |
| $H_2O/H_2$ mol/mol (reactor inlet) | 0.3 | 0.0 | 0.3 |
| Ethane Yield on Olefin, Wt% | 10.7 | 48.6 | 11.7 |
| $C_5+$ Yield on Olefin, Wt% | 62.7 | 16.8 | 62.9 |
| $C_2$ = Conversion | 90.1 | 99.9 | 95.5 |
| Average Reactor Temperature °C/(°F) | 285(544) | 297(567) | 299(570) |
| RI (reaction severity index) | 1.43 | 67.75 | 2.54 |

These results show that catalyst selectivity changes are reversible as water is removed from and returned to the reactor.

**Claims**

1. A catalytic process for converting an ethene-containing lower olefinic feedstock to a heavier liquid hydrocarbon product, comprising the step of:
contacting the ethene-containing feedstock at an elevated temperature with at least one conversion catalyst comprising a nickel ethene oligomerization component and a shape-selective medium pore acidic zeolite component to convert at least a portion of the lower olefinic component to heavier hydrocarbons, water being cofed with said feedstock during the contacting step thereby maintaining the nickel component in an oxidized state.

2. The process of claim 1 wherein the feedstock comprises at least 5 mol% ethene.

3. The process of claim 1 or claim 2 wherein the feedstock contains a reducing gas.

4. The process of claim 3 wherein the feedstock reducing gas is hydrogen.

5. The process of claim 4 wherein the water is cofed in a molar rate of at least 0.1 moles per mole of hydrogen.

6. The process of any preceding claim wherein the zeolite component has a silica-to-alumina molar ratio greater than 12 and a constraint index of about 1 to 12.

7. The process of claim 6 wherein the zeolite component is ZSM-5.

8. The process of any preceding claim wherein the nickel component is present at least in part as ion-exchanged nickel on the zeolite component.

9. The process of any preceding claim wherein said elevated temperature is 100–450°C.

## Patentansprüche

1. Katalytisches Verfahren zur Umwandlung eines Ethen enthaltenden, niederen, olefinischen Ausgangsmaterials zu einem schwereren, flüssigen Kohlenwasserstoffprodukt, welches die Schritte umfaßt: Kontakt des Ethen enthaltenden Ausgangsmaterials bei erhöhter Temperatur mit mindestens einem Umwandlungskatalysator, der eine Ethenoligomerisations-Nickelkomponente und eine formselektive, saure Zeolithkomponente mit mittleren Poren umfaßt, um mindestens einen Teil der niederen, olefinischen Komponente in schwerere Kohlenwasserstoffe umzuwandeln, wobei Wasser während des Kontaktschritts gleichzeitig mit dem Ausgangsmaterial zugeführt wird, wodurch die Nickelkomponente im oxidierten Zustand bleibt.

2. Verfahren nach Anspruch 1, worin das Ausgangsmaterial mindestens 5 Mol-% Ethen umfaßt.

3. Verfahren nach Anspruch 1 oder 2, worin das Ausgangsmaterial ein reduzierendes Gas enthält.

4. Verfahren nach Anspruch 3, worin das reduzierende Gas des Ausgangsmaterials Wasserstoff ist.

5. Verfahren nach Anspruch 4, worin das Wasser mit einer Molmenge von mindestens 0,1 Mol pro Mol Wasserstoff gleichzeitig zugeführt wird.

6. Verfahren nach einem der vorstehenden Ansprüche, worin die Zeolithkomponente ein Siliciumdioxid/Aluminiumoxid-Molverhältnis von mehr als 12 und einen Zwangsindex von 1 bis 12 aufweist.

7. Verfahren nach Anspruch 6, worin die Zeolithkomponente ZSM-5 ist.

8. Verfahren nach einem der vorstehenden Ansprüche, worin die Nickelkomponente zumindest teilweise als durch Ionenaustausch eingebrachtes Nickel auf der Zeolithkomponente vorhanden ist.

9. Verfahren nach einem der vorstehenden Ansprüche, worin die erhöhte Temperatur 100 bis 450°C beträgt.

## Revendications

1. Un procédé catalytique de conversion d'une charge d'alimentation oléfinique inférieure contenant de l'éthène en un produit hydrocarbure liquide plus lourd, comprenant l'étape de:
   – mise en contact, à une température élevée, de la charge d'alimentation contenant de l'éthène avec au moins un catalyseur de conversion comprenant un dérivé d'oligomérisation nickel-éthène et une zéolite sélective de forme à pores moyens pour convertir au moins une partie du dérivé oléfinique inférieur en hydrocarbures plus lourds, de l'eau étant co-alimentée avec ladite charge alimentaire au cours de l'étape de mise en contact, maintenant par la même le dérivé nickel à l'état oxydé.

2. Le procédé selon la revendication 1, dans lequel la charge d'alimentation comprend au moins 5 moles % d'éthène.

3. Le procédé selon la revendication 1 ou la revendication 2, caractérisé en ce que la charge d'alimentation contient un gaz réducteur.

4. Le procédé selon la revendication 3, caractérisé en ce que le gaz réducteur de la charge d'alimentation est de l'hydrogène.

5. Le procédé selon la revendication 4, caractérisé en ce que l'eau est co-alimentée selon un rapport molaire d'au moins 0,1 mole par mole d'hydrogène.

6. Le procédé selon l'une quelconque des revendications précédentes, caractérisé en ce que le composé zéolitique a un rapport molaire silice/alumine supérieur à 12 et un indice de contrainte d'environ 1 à 12.

7. Le procédé selon la revendication 6, caractérisé en ce que le composé zéolitique est une ZSM-5.

8. Le procédé selon l'une quelconque des revendications précédentes, caractérisé en ce que le dérivé nickel est présent au moins en partie en tant que nickel à ion échangé sur le composé zéolitique.

9. Le procédé selon l'une quelconque des revendications précédentes, caractérisé en ce que ladite température élevée est de l'ordre de 100 à 450°C.

FIG. 1

FEED GAS

OFF GAS

LIGHT GASOLINE

LIQUID H₂O

MAKE-UP H₂O

EXCESS CONDENSATE

HEAVY GASOLINE

EXCESS CONDENSATE

EP 0 256 707 B1

# FIG. 2

### ETHYLENE CONVERSION

○  0.9 WT. % Ni-ZSM-5, N$_2$ PRETREAT
●  0.9 WT. % Ni-ZSM-5, H$_2$ PRETREAT
□  HZSM-5, H$_2$ PRETREAT

FIG. 3

FIG. 4

FIG. 5

1% NI/ZSM-5 W/H₂O CO-FEED
C₂= FEED
450 PSIG

NO H₂O AT RXT INLET

0.1 MOL H₂O/MOL H₂ AT RXT INLET

0.3 MOL H₂O/MOL H₂ AT RXT INLET

ETHANE YIELD ON FEED OLEFIN, WT%

ETHYLENE CONVERSION

EP 0 256 707 B1